(19)

Europäisches
Patentamt
European
Patent Office
Office européen
des brevets

(11) **EP 2 353 506 A1**

(12) **EUROPEAN PATENT APPLICATION**
published in accordance with Art. 153(4) EPC

(43) Date of publication:
**10.08.2011 Bulletin 2011/32**

(21) Application number: **09824448.6**

(22) Date of filing: **06.11.2009**

(51) Int Cl.:
***A61B 5/145*** (2006.01)

(86) International application number:
**PCT/ES2009/000529**

(87) International publication number:
**WO 2010/052354 (14.05.2010 Gazette 2010/19)**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **07.11.2008 ES 200803183**

(71) Applicant: **Sabirmedical, S.L.
08028 Barcelona (ES)**

(72) Inventors:
• **RIBAS RIPOLL, Vicente Jorge
E-08028 Barcelona (ES)**

• **GARCÍA LLORENTE, Víctor Manuel
E-08028 Barcelona (ES)**
• **MONTE MORENO, Enrique
E-08034 Barcelona (ES)**

(74) Representative: **ZBM Patents
Zea, Barlocci & Markvardsen
Plaza Catalunya, 1
08002 Barcelona (ES)**

(54) **SYSTEM AND APPARATUS FOR NON-INVASIVE MEASUREMENT OF GLUCOSE LEVELS IN BLOOD**

(57) A system for estimating the glucose levels in blood is developed in the present invention. Said system establishes a physiological model of the pulse wave and its energy, which are also correlated with the glucose metabolic function, for generating a fixed length vector containing the values of the previous model combined with other variables related to the user such as, for example, age, sex, height, weight, etc... This fixed length vector is used as an excitation of a function estimation system based on "random forests" for the calculation of the interest variable. The main advantage of this parameter estimation system lays in the fact that it does not apply any restriction a priori on the function to be estimated, and that it is robust in front of heterogeneous data, such as in the case of the present invention.

FIG.2

**Description**

**FIELD OF THE INVENTION**

The present invention develops a system for non-invasive measurement of the glucose levels in blood, independent from the current methods based on blood sample analysis (Oxidasa Glucose molecule reduction) or by means of an absorption spectrum analysis of glucose in blood. With this purpose, a new method is presented based on the function approximation by means of random forests implemented by means of a DSP or FPGA device, whose input is a pre-processed version of the plethysmographic pulse combined with other variables from the patient.

**BACKGROUND OF THE INVENTION**

Diabetes Mellitus (DM) comprises a group of metabolic disorders, which share a hyperglycaemic phenotype, (increase of blood glucose levels in patients). Several types of DM exist, which are a result of a complex interaction between genetic factors, and environmental factors and lifestyle (sedentary, diet, etc...). Depending on the causes of DM, factors that contribute to hyperglycemia may include the reduction of insulin hormone secretion, insufficient use of glucose at the metabolic level, or an increased production of glucose by the body.

Disorders associated with DM seriously compromise the body. Also, such disorders are a major economic burden to the healthcare system. In developed countries, DM is the primary cause of kidney failure, non-traumatic amputation of the lower extremities, and blindness in adults. In fact, studies have shown that approximately 1.7% of the world's population suffers from DM, and that this percentage is likely to increase in the medium and long term, thus, DM remaining a major cause of morbidity and mortality.

The protocols published by the World Health Organization (WHO) define the following diagnose criteria of the DM:

- Diabetes symptoms with a glucose concentration in a random sample of blood greater than 11 mmol/L or 200 mg/dL or,
- Blood plasma glucose (after fasting) above 7.0 mmol/L or 126mg/dL or
- Blood plasma glucose (two hours) greater than 11.1 mmol/L or 200 mg/dL during the glucose tolerance oral test.

Currently, measuring glucose levels involves taking a blood sample during the testing process. Various devices exist for determining glucose levels in diabetic patients, based on the reduction of the reagent glucose oxidase (GOX). Such devices use a small blood sample, obtained with a lancet, and deposited on a small test strip impregnated with GOX. Glucose in the blood reacts with GOX, and hydrogen peroxide (H2O2) is obtained as a result. The amount of hydrogen peroxide causes a change in the impedance of the strip, which correlates with the level of glucose in the blood.

Said systems are highly invasive, because they require patients with diabetes to puncture their fingers up to four times a day to obtain blood samples and monitor their glucose levels.

With the aim to eliminate the hurting related to the puncturing and to minimize the sources of infection, systems exist that utilize spectroscopic techniques to measure glucose levels means of spectroscopy (emission, transmission, and reflection methods). These systems are adversely affected by water in the body, a low glucose concentration, and optical effects produced by the skin, and are thus unreliable. In fact, nowadays there is no known commercial device which uses such techniques.

Patent 4704029 establishes a system for monitoring the glucose in blood for controlling an insulin bomb for a domestic use. Said glucose monitoring uses an optical refraction device, which measures the refraction index of the light over a transparent interface (light quantity reflexed near the critic angle).

Patent 5137023 describes non-invasive system for the measurement of the glucose concentration in an absorption matrix. The system directs light rays towards the matrix using wavelengths sensitive to glucose (absorption or reflection) in combination with other wavelengths not affected by said substance. Said patent also uses the photoplethysmography principle for measuring the change in the light intensity of the light on the absorption matrix before and after the blood volume changes during the systolic phase of the cardiac cycle. Said change in the light intensity, is used to adjust the light intensity to measure the glucose concentration.

Patents 5361758 and US6928311 B1 describe a non-invasive system for monitoring the glucose levels in blood and tissues using a polychromatic source of light, which emits in a wide wavelength spectrum in the range near infrared (NIR). Said light passes through the finger or ear and is separated on its main components by means of defraction or refraction. A microprocessor uses said components to calculate the quantity of light transmitted and calculate the medium absorbency. Said system calculates the second derivative of the measurements and performs a linear regression for estimating the glucose concentration.

Patent 5383452 measures the glucose concentration in blood using the polarization changes in the emitted light caused by the chromophores and particles dissolved in the blood. Said system requires a calibration by means of conventional methods (for example, a blood sample analyzed by means of the technique known as Glucose Oxidasa).

Patent 5692504 performs a determination of the glucose concentration in blood measuring the light interaction with a biological matrix. Since the light properties change based on the substances present in the matrix, it is possible to correlate said changes with the glucose concentration to be estimated.

Patent US6526298B1 presents a method for determining the glucose in blood by means of a calculation of optical parameters (reflection and refraction of light) on several temperatures.

Patent US7356364B1 presents a device for monitoring the glucose in blood by means of the modulation of a LASER emitter and the calculation of the absorption spectrum of said light. Said system can be used in a non-invasive form or by means of an implant.

Patents US2001/0039376A1 and US2004f/0127779A1 present a system for measuring the glucose concentration in blood by means of the transmission of one or more wavelengths from which the absorption/attenuation is measured, for estimating the oxygen level in blood. Said invention uses an extra wavelength at 1060nm (glucose concentration sensitive) to measure the sugar concentration in blood from the light absorption at that wavelength.

Patent US2003/0225321A1 presents a system for non-invasive measurement of glucose levels in blood by means of the transmission of several wavelengths. Said wavelengths are transmitted through the eye's humour of the patient and the reflected light is detected and analyzed (changes in the emitted wavelength's polarization) to provide an estimation of glucose concentration.

Patent US2005/0070770A1 describes a system and apparatus for measuring glucose concentration in blood based on the interaction of light with several components dissolved in the blood by means of a differential analysis.

Patent US2006/0149142A1 presents a system and apparatus for measuring the glucose concentration by means of the light transmission on a determined wavelength through a tissue and calculating the difference between the propagation paths of said light. Said invention performs an estimation of glucose levels from a linear regression model related with the optical characteristics of the tissue and the difference between paths previously described.

However, it is still to be solved the need to establish a robust, reliable, fast and safe system for determining the levels of glucose concentration, which captures the metabolic complexity of the glucose levels in blood without imposing any restrictions a priori in a functional level over the analyte to be estimated.

## BRIEF SUMMARY OF THE INVENTION

According to the priori art of the invention previously detailed, the proposed system and apparatus of the present invention is based in the inference of the functional relationship between the shape of the pulse (PPG) and the glucose levels in blood wherein the information is deduces from the dependence between the shape of the pulse and its statistics with the state of the glucose of the patient.

The input information for performing the estimation of the glucose level in blood is processed to ease the work of the function estimator. Since the PPG signal has a variable duration, a treatment is performed to generate a fixed length vector for each measurement. This vector contains information about the shape of the pulse (auto-regression coefficients and mobile average), average distance between pulses, its variance, information about the instantaneous energy, energetic variation and clinical information of the person, such as, for example, sex, age, weight, height, clinical information of the patient (body mass index or similar measurements), etc...

The system for the inference of functions works blindly in the sense that no functional restriction is imposed to the relationship between the pulse and the glucose level in blood. Since the functional form which relates the PPG with the glucose levels is unknown, a system for infering said function has been chosen which is robust with irrelevant input variables such as clinical information and parameters derived from the shape of the PPG wave. Furthermore, said technique is related with other parameters as previously described in the prior art of the present invention. The preferred system for estimating functions in the present invention are the "random forests", in comparison to other machine learning systems and pattern recognition such as, for example, decision and regression trees (CART), Splines, classifier comitees, Support Vector Machines and Neuronal networks. The random forests are based in the parallel generation of a set of decision trees, which estimate the function with a random selection of variables in each node not performing the prune of the nodes, and training each tree with a random subset of the training database in such a way that each tree has a different generalization systematic error. Therefore, when performing an average of the estimations of each tree, the systematic error are compensated and the estimation variance decreases.

The implementation of the present invention comprises two different phases. First phase is the training of the system, which is performed only once and, therefore, it does not require any calibration/personalization afterwards. This phase consists of the obtaining of a database with information about several patient's parameters including, sex, weight, age, etc. combined with a recording of the plethysmographic wave. This information is used in the estimation of the parameters of the decision trees and are stored in the system.

The second phase consists of loading the information of the set of trees obtained in the training phase and recording the plethysmographic wave of the patient in the measurement moment with other variables such as, for example, sex, weight, age, etc... In this phase, the system reads the information of the plethysmographic pulse, performs its processing and generates a fixed length vector with the information that describes the signal. The additional information of the person is added to this vector and a set of random forests is applied, which calculate several intermediate functions of the variable of interest. Afterwards, the glucose level in blood is calculated from said intermediate functions.

**BRIEF DESCRIPTION OF THE DRAWINGS/FIGURES**

Figure 1 of the present invention shows a waveform of the pulse obtained by means of an invasive catheterism.
Figure 2 of the present invention shows a general block diagram of the described system and method.
Figure 3 of the present invention shows a plethysmographic waveform obtained by means of a digital pulse-oximeter.
Figure 4 shows a detailed block diagram of the pre-processing system described in the present invention.
Figure 5 shows a detailed block diagram of the AR filter for establishing the stochastic model of the pressure pulse physiology described in the present invention.

**DETAILED DESCRIPTION OF THE INVENTION**

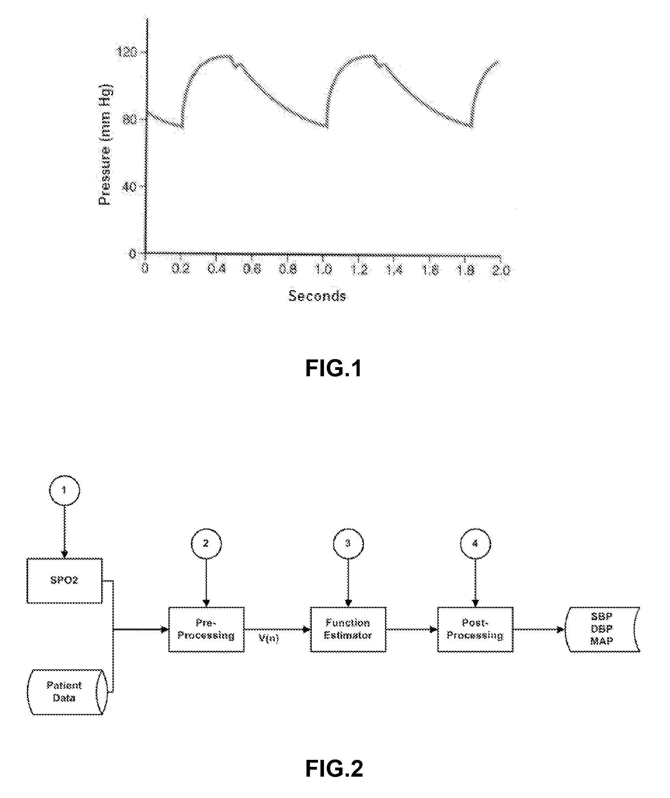

The present invention consists of a system for monitoring glucose in blood (figure 2) its data being evaluated by means of a device (1) for capturing plethysmographic signals (optical, acoustic or mechanic), the preferred embodiment the invention consisting of a pulsioximetry system (SpO2). The PPG information is combined with other data of the patient such as, for example, age, sex, height, weight, etc. and is analyzed with a digital pre-processing system (2), which simplifies the subsequent processing and estimation of the variable of interest (glucose concentration in blood). Said system captures in a better way all the parameters which affect in a random way the metabolic management of the glucose of the patient.

The vector of the obtained stochastic model is linked with a digital system (3) which approximates functions based on "random forests", its main function being estimating the glucose concentration with several functions related with that, with the object of decreasing the estimation error in the post-processing step (4). The main function of the system (4) is to estimate the final values of the glucose concentration by means of the averaging of the functions of the previous step (3) to decrease the systematic error (bias) and the variance of the estimations performed with said concentration. Systems (2, 3, and 4) are implemented by means of a FPGA or DSP device.

The system (1) for obtaining the PPG wave implements a simple technique, non invasive and low cost for detecting the volume changes in the micro-vascular network of a tissue. The most basic implementation of said system requires few opto-electronic components including:

1. One or more sources for illuminating the tissue (for example, the skin)
2. One or more photo-detectors for measuring the little variations in the intensity of light associated with the changes in the infusion of the tissue in the detection volume.

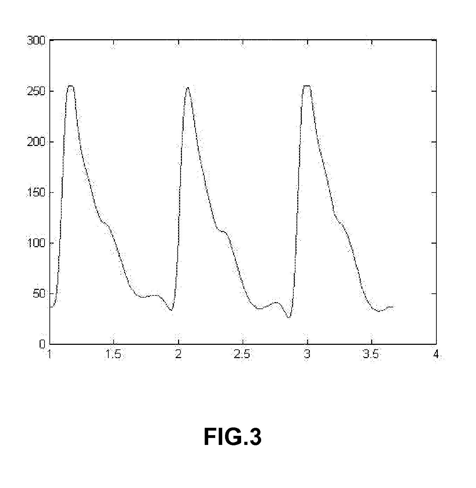

The PPG is normally used in a non-invasive way and it operates in the infrared or near infrared (NIR) wavelengths. The most known PPG waveform is the peripheral pulse (figure 3) and it is synchronized with each heartbeat. At this point it is important to take notice the similarity between the obtained waves by means of PPG and the obtained pulses by means of invasive catheterism (figures 1 and 3). Because of the information of great value obtained by means of PPG, it is considered one of the main features of the present invention.

The PPG wave comprises a physiological pulsed wave (AC component) attributed to the blood volume changes synchronized with each heartbeat. Said component is superimposed with another fundamental component (DC component) related to the respiratory rhythm, the central nervous system's activity, thermo-regulation and metabolic function. The fundamental frequency of the AC component is found around 1Hz depending on the cardiac rhythm (figure 3).

The interaction between the light and the biological tissues is complex and includes optical processes like the scattering, absorption, reflection, transmission and fluorescence. The selected wavelength for the system (1) is highly important because of the following:

1. Water's optical window: the main constituent of tissues is water. It highly absorbs the light with ultraviolet wavelengths and long wavelengths within the infrared band. A window exists in the water's absorption spectrum which allows the passing of visible light (red) or NIR easier through the tissue and allowing the measure of the blood flux or its volume within this wavelengths. Therefore, the present invention uses NIR wavelengths for system (1).

2. Isobestic wavelength: substantial differences exist related to the oxyhemoglobin (HbO2) and the reduced hemoglobin (Hb) except for this wavelength. Therefore, the signal will not be affected by the changes in oxygen saturation in the tissue in this wavelength (i.e. near 805 nm, for the NIR range).

3. Tissue penetration: the penetration's depth of light in a tissue for a determined radiation intensity is also a function of the selected wavelength. For PPG, the penetration's volume (depending on the probes being used) is of the order of 1 cm3 for transmission systems like the one used in (1).

The PPG pulse (figure 3) presents two different phases: the anacrotic phase, which represents the rise of the pulse, and the catacrotic phase, which represents the fall of the pulse. The first phase is related to the cardiac systole while the second is related to the dystole and the reflections of the wave in the periphery of the circulatory system. In the PPG, a diacrotic pulse is also usually found in the catacrotic phase in healthy patients and without arteriosclerosis or arterial rigidity.

As it has previously been described in the prior art of the present invention, the propagation of the pressure pulse PP along the circulatory tree also has to be taken into account. Said PP changes its shape while it moves towards the periphery of the circulatory tree suffering amplifications/attenuations and alterations of its shape and temporary characteristics. These changes are caused by the reflections suffered by the PP because of the narrowing of the arteries in the periphery. The PP pulse propagation in the arteries is further complicated by a phase distortion depending on the frequency.

Because of this, the representations of PP by means of ARMA stochastic models (Auto-Regressive Moving Average) and by means of the Teager-Kaiser coupled to an AR system (2) have been considered.

As shown in figures 1 and 3, the PP is similar to the PPG, wherein similar changes are observed during vascular pathologies (cushioning caused by stenosis or change in the pulse).

The pulse-oxymeter of the system (1) uses the PPG to obtain information about the oxygen saturation (SpO2) in the arteries of the patient. As previously described, the SpO2 can be obtained by illuminating the tissues (normally a finger or an ear's lobe) in the red and NIR wavelengths. Normally, the SpO2 devices use the commutation between both wavelengths to determine said parameter. The amplitudes of both wavelengths are sensitive to the changes in SpO2 because of the absorption difference of HbO2 and Hb in those wavelengths. The SpO2 may be obtained from the ratio between amplitudes, the PPG and the AC and DC components.

In pulse oximetry, the intensity of the light (T) transmitted through the tissue is commonly referred to as a DC signal. The intensity is a function of the optical properties of tissue (i.e. the absorption coefficient $\mu_a$ and the scattering coefficient $\mu'_s$). Arterial pulsation produces periodic variations in the concentrations of oxy and deoxy hemoglobin, which may result in periodic variations in the absorption coefficient.

The intensity variations of the AC component of the PPG may be expressed in the following way:

$$\mathrm{AC} = \Delta\mathrm{T} = \frac{\partial T}{\partial \mu_a}\Big|_{\mu_a,\mu'_s} \Delta\mu_a \quad (\mathrm{I})$$

This physiological waveform is proportional to the variation of light intensity, which, in its turn, is a function of the absorption and scattering coefficients ($\mu_a$ and $\mu'_s$, respectively). The component $\Delta\mu_a$, may be written as a linear variation of the concentrations of oxy and deoxy hemoglobin ($\Delta c_{ox}$ and $\Delta c_{deox}$) as follows:

$$\Delta\mu_a = \varepsilon_{ox}\Delta c_{ox} + \varepsilon_{deox}\Delta c_{deox} \quad (\mathrm{II})$$

Being $\varepsilon_{ox}$ and $\varepsilon_{deox}$ the extinction coefficients (i.e. fraction of light lost as a result of scattering and absorption per unit distance in a particular environment). Based on these equations, the arterial oxygen saturation (SpO2) may be determined by:

$$SpO_2 = \frac{\Delta c_{ox}}{\Delta c_{ox} + \Delta c_{deox}} \qquad (III)$$

The expression of SpO2 as a function of the AC component may be obtained by the direct application of equations (I) and (III) at selected wave-lenghts (red and NIR).

$$SpO_2 = \frac{1}{1 - \dfrac{x\varepsilon_{ox}(NIR) - \varepsilon_{ox}(R)}{x\varepsilon_{deox}(NIR) - \varepsilon_{deox}(R)}} \qquad (IV)$$

wherein,

$$x = \frac{\left.\dfrac{\partial T(NIR)}{\partial \mu_a}\right|_{\mu_a,\mu'_s}}{\left.\dfrac{\partial T(R)}{\partial \mu_a}\right|_{\mu_a,\mu'_s}} \frac{AC(R)}{AC(NIR)} \qquad (V)$$

Normalizing the AC component with the DC to compensate the low frequency effects which are unrelated to the synchronous changes in the blood, the following is obtained:

$$R = \frac{\dfrac{AC(R)}{DC(R)}}{\dfrac{AC(NIR)}{DC(NIR)}}$$

Including this parameter in (IV) yields:

$$SpO_2 = \frac{1}{1 - \dfrac{kR\varepsilon_{ox}(NIR) - \varepsilon_{ox}(R)}{kR\varepsilon_{deox}(NIR) - \varepsilon_{deox}(R)}} \qquad (VI)$$

being:

$$k = \frac{\dfrac{\Delta T(NIR)}{DC(NIR)}}{\dfrac{\Delta T(R)}{DC(R)}}$$

Wherein $\Delta T(NIR)$ and $\Delta T(R)$ correspond to equation (I), evaluated at R and NIR wavelengths.
Although the equation (VI) is an exact solution for SpO2, k cannot be evaluated since it doesn't have $T(\mu_a,\mu'_s)$. However, k and R are functions of the optical properties of the tissue, being possible to represent k as a function of R.

More specifically, it may be possible to express k as a linear regression with the following form:

$$k = \mathrm{a}R + b \qquad \text{(VII)}$$

15 This linear regression implies a calibration factor empirically derived but assuming a flat wave with intensity P, its absorption coefficient may be defined as:

$$dP = \mu_a P dz \qquad \text{(VIII)}$$

where dP represents the differential change in the intensity of a light beam passing through an infinitesimal dz in a homogeneous medium with an absorption 20 coefficient of $\mu_a$. Therefore, integrating over z the Beer-Lambert law is obtained.

$$P = P_0 e^{\mu_a Z} \qquad \text{(IX)}$$

Assuming that $T \approx P$, equation (VII) is thus reduced to k=1, which is the preferred approximation in the pulse-oximetry measurement performed in the present invention.

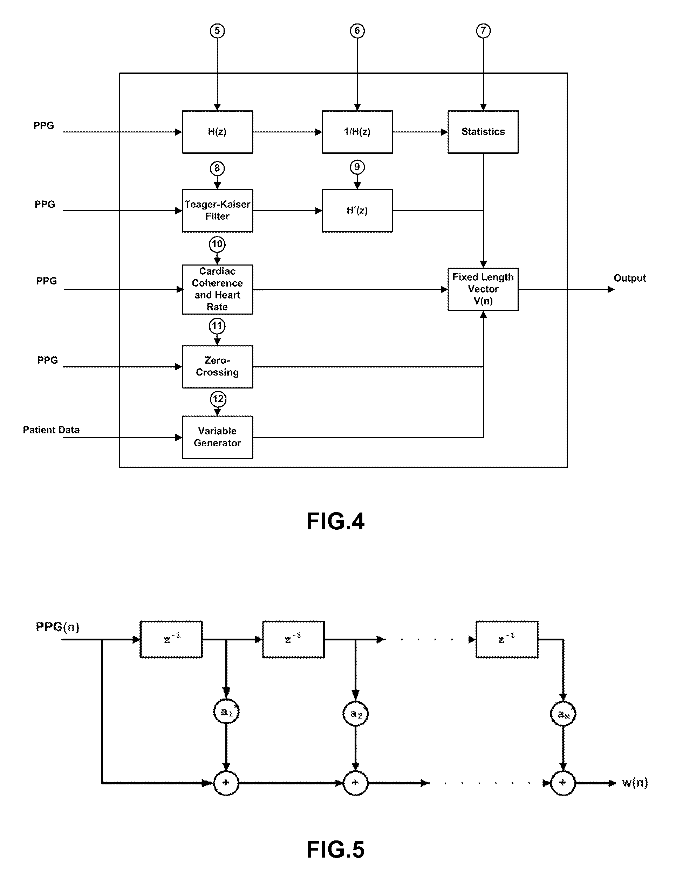

The PPG signal obtained by the system (1) is used as the system's excitation (2) (figure 4) of the present invention, its main function being performing a pre-processing, which simplifies the functions to estimate.

Several parameters exist, which are basic in the form and in the propagation of the pressure pulse (PP). Said parameters are related with the cardiac output, heart rate, cardiac synchrony, breathing rate, and the metabolic function. I has also been previously detailed the close relationship between the PP and the PPG. Therefore, since the previously detailed parameters are important in the shape and propagation of the PP, the parameters listed above may also influence the PPG signal.

According to the above, the preferred embodiment of the present invention uses a stochastic ARMA(q,p) modeling (auto-regressive moving average model of order q (MA) and p(AR)).

By definition, the time series PPG(n), PPG(n-1),..., PPG(n-M) represents the realization of an AR process of order p=M if it satisfies the following finite difference equation (FDE):

$$PPG(n) + a_1 PPG(n-1) + \cdots + a_M PPG(n-M) = w(n) \qquad \text{(X)}$$

Wherein the coefficients $[a_1, a_2,...,a_M]$ are the AR parameters and w(n) is a white process. The term $a_k PPG(n-k)$ is the inner product of the $a_k$ coefficient and PPG$(n-k)$, wherein k=1, .., M. The equation (X) may be rewritten as the following:

$$PPG(n) = v_1 PPG(n-1) + v_2 PPG(n-2) + \cdots + v_M PPG(n-M) + w(n) \qquad \text{(XI)}$$

wherein $v_k = -a_k$.

From the above equation, it follows that the current pulse value PPG(n) equals a finite linear combination of the above values (PPG$(n-k)$) plus a prediction error term $w(n)$. Therefore, rewriting the equation (X) as a linear convolution, it is obtained:

$$\sum_{k=0}^{M} a_k \mathrm{PPG}(n-k) = w(n) \qquad \text{(XII)}$$

It can be defined that $a_0 = 1$ without loss of generality, and thus, the Z-transform of 10 the predictive filter may be given by:

$$A(z) = \sum_{n=0}^{M} a_n z^{-n} \qquad \text{(XIII)}$$

Defining PPG z as the Z-transform of the PPG pulse, then:

$$A(z)\mathrm{PPG}(z) = W(z) \qquad \text{(XIV)}$$

where

$$W(z) = \sum_{n=0}^{M} v(n)z^{-n} \qquad \text{(XV)}$$

Figure 5 shows the analysis filter of the AR component of the PPG(n) pulse obtained by the system (1).
In the MA (moving average) component case of order q=K of the PPG(n) pulse, it can be described as the response of a linear discrete filter (filter with all zeros) excited by a Gaussian white noise. Thus, the MA response of said filter written as an EDF may be:

$$\mathrm{PPG}_{\mathrm{MA}}(n) = e(n) + b_1 e(n-1) + \cdots + b_K e(n-K) \qquad \text{(XVI)}$$

wherein $[b_1, b_2, ..., b_K]$ are the constants called MA parameters and $e(n)$ is a white noise process of zero mean and variance $\sigma^2$. Therefore, relating equations (XII) and (XVI) we obtain the following:

$$\mathrm{PPG}(n) = e(n) + \sum_{k=0}^{p} a_k \mathrm{PPG}(n-k) + \sum_{k=0}^{q} b_k e(n-k) \qquad \text{(XVII)}$$

Being $e(n)$ the error terms of the ARMA(q,p) model. Taking the Z transform of the above equation in (XVII) the following is obtained:

$$\mathrm{PPG}(z) = \frac{1 + B(z)}{1 - A(z)} E(z) \qquad \text{(XVIII)}$$

Since the first terms of the AR and MA vectors may be equal to 1 without loss of generality, the expression of the ARMA(q,p) model (5) in the system (2) may be given by:

$$H(z) = \frac{B(z)}{A(z)} \qquad \text{(XIX)}$$

5 Being A(z) and B(z) the AR and MA components of PPG(n) respectively. The preferred embodiment of the present invention uses an ARMA model of order q=1 and p=5, although any order of p and q in a range between [4,12] can be used

Once the ARMA (q,p) is calculated, by means of the Wold decomposition and the Levinson-Durbin recursion, the input signal is filtered with an H(z) inverse filter (6). Also, the statistics of the residual error e(n) are calculated with the subsystem (7).

The information obtained from these subsystems is stored in the output vector V(n) of a fixed dimension

The pre-processing system (2) of the present invention also comprises a subsystem (8) which calculates the Teager-Kaiser operator and models its output by means of an AR process of p order which is equivalent to the previously described.

In this case, without a loss of generality, the PPG pulse may be considered as a signal modulated in both amplitude and frequency, or an AM-FM signal, being the type of:

$$\mathrm{PPG}(t) = a(t)\cos \int_{0}^{t} w(\tau(\tau) \qquad \text{(XX)}$$

Being a(t) and w(t) the instantaneous amplitude and frequency of the PPG. The Teager-Kaiser operator of a determined signal is defined by:

$$\Psi[x(t)] = [x'(t)]^2 - x(t)x''(t) \qquad \text{(XXI)}$$

wherein $x'(t) = \dfrac{\mathrm{d}x(t)}{\mathrm{d}t}$ .

This operator applied to the AM-FM modulated signal of equation (XX) results in the instantaneous energy of the source that produces the oscillation of the PPG. That is:

$$\Psi[\mathrm{PPG}(t)] \approx a^2(t)w^2(t) \qquad \text{(XXII)}$$

wherein the approximation error is negligible if the instantaneous amplitude a(t) and the instantaneous frequency w(t) do not vary too fast with respect to the average value of w(t); as is the case of the PPG pulse for the estimation of glucose levels in blood.

The AR process of order p of $\Psi[\mathrm{PPG}(t)]$ is implemented with a filter (9) equivalent to that of figure 5. The preferred embodiment of the present invention uses an AR model of order p=5, although any order of p comprised between 4 and 12 may be used.

Once the stochastic models based on an ARMA(q,p) model (5, 6 and 7) and the ARMA(q,p) model over the Teager-Kaiser operator (8 and 9), the present invention calculates the heart rate (HR) and cardiac synchrony (for example, heart rate variability), from the PPG signal by means of subsystem (10). The preferred embodiment of the present invention calculates the heart rate (HR) over time windows of the PPG which may vary between 2 seconds and 5 minutes.

The pre-processing system (2) comprises also a subsystem (11), which calculates the zero crossings of the PPG signal input, as well as the variances of these zero crossings. The preferred embodiment of the present invention calculates the heart rate over time windows of the PPG which may vary between 2 seconds and 5 minutes.

Finally, the pre-processing system (2) comprises a subsystem (12) for the generation of variables related with the patient under study which include:

1. Sex, age, weight, height, if he has eaten any food, time of the day.
2. Body mass index.
3. Weight divided by age.
4. Weight divided by HR.
5. Height divided by HR.
6. HR divided by age.
7. Height divided by age.
8. Age divided by body mass index.
9. HR divided by body mass index.
10. Blood pressure.

All the obtained data in the subsystems comprised in the system (2) are stored in the output fixed length vector V(n).

Once the features vector of fixed length V(n) is obtained, an estimation of the SBP, DBP and MAP may be performed by means of the function approximation system (3) based in "random forests". The function estimation system presented in this invention has the advantage of not requiring any calibration once the 'random forest' has been correctly trained.

In a specific way, a random forest is a classifier which consists of a set of classifiers with a tree structure $\{h(V, \Theta_k), k = 1, ...\}$ wherein $\Theta_k$ are independent random vectors and identically distributed (i.i.d.), wherein each vector deposits a single vote for the most popular class of the input V. This approximation presents a clear advantage related to the reliability over other classifiers based on a single tree, in addition to not imposing any functional restrictions on the relationship between the pulse and glucose levels in the blood.

The random forests used in the present invention are generated by growing decision trees based on the random vector $\Theta_k$, such that the predictor $h(V, \Theta)$ takes numeric values. This random vector $\Theta_k$ associated to each tree provides a random distribution on each node and, at the same time, it also provides information on the random sampling of the training base, resulting in different data subsets for each tree. Based on the result, the generalization error of the classifier used may be provided by the following:

$$PE = E_{V,Y}(Y - h(V))^2 \qquad \text{(XXIII)}$$

Since the generalization error of a random forest is smaller than the generalization error of a single decision tree, defining

$$Y - h(V, \Theta) \qquad \text{(XXIV)}$$

$$Y - h(V, \Theta') $$

yields

$$PE(\text{forest}) \leq \rho PE(\text{tree}) \qquad \text{(XXV)}$$

Each tree has a different generalization error and *p* represents the correlation between the residuals defined by equation (XXIV). Thus, a lower correlation between residuals may result in better estimates. In the present invention, this minimum correlation is determined by the random sampling process of the feature vector at each node of the tree that is being trained in the subsystem (2). To further decrease the generalization error, the present invention estimates both the parameter of interest (glucose levels in blood) and linear combinations of the previously discussed parameters (height, weight, age, gender, etc.).

The random forests consist of a set of CART-type decision trees (Classification and Regression Trees), altered to introduce systematic errors (XXV) on each one and afterwards, by means of a bootstrap system, a systematic variations (these two processes are modeled by the parameter Θ in the analysis of the predictor h(V, Θ). The systematic error in each embodiment is introduced by two mechanisms:

1. Random choice at each node in a subset of attribute, which does not allow to establish a statistical equivalence among the partitions made between similar nodes in different trees such that each tree behaves differently.

2. The trees are allowed to grow up until their maximum. In this case, trees act similarly to a lookup table based on rules. Because of the sampling of the attributes, these are lookup tables to search with different structures.

The result of the above process is that each tree has a different systematic error.

Also, for each of these modifications, each tree is trained with "bootstrap" type samples (for example, a sample of input data is taken, leading to a fraction of the input data missing while another fraction of the data is duplicated). The effect of the bootstrap samples is that variability is introduced, which when performing the average of the estimations, it is compensated.

The overall result of the above features is system (4), in the systematic error and variability in the error can be easily compensated resulting more precise than other type of function estimators (XXV). In this system, the base classifier is a tree, which decides on the basis of levels, which makes it robust when presented with input distributions which include outliers or heterogeneous type data (as in the present invention).

The preferred embodiment of the system (4) consists of taking random samples of two elements of 47 in a node level (which can be implemented in variations between 2 and 47) and a bootstrap size of 100, also with the possibility of varying between sizes of 25 and 500.

The hand-held device according to the invention may incorporate a display for displaying data and commands for controlling the operation of the device. It also comprises at least an acoustic, mechanical, and/or optical probe, whose signals are interpreted by a post-processing system by means of a CPU centralized by means of DSP, FPGA or micro controllers. It also comprises working memories for storing the data and operative processes of the system.

The manual device of the invention may also comprise manual control buttons, according to the prior art, to activate and control it, plus batteries and/or access to an external power source.

Finally, the results obtained by the present invention may be transmitted to a PC to be analyzed, via serial port or USB or a network connection, for example by means of WiFi or Bluetooth.

It has to be noted that any alterations of the details or shape of the invention are comprised within the essence of the invention.

**Claims**

1. System and apparatus for a non-invasive measurement of glucose levels in blood, **characterized in that**:

   - they incorporate an autoregressive mobile average type (ARMA) pre-processing step (5), (6) and (7) on the input signal
   - they incorporate a stochastic model of the autoregressive mobile average type (ARMA) blood pressure pulse on the Teager-Kaiser operator of the input signal (8) and (9).
   - they incorporate clinical data (sex, age, height, body mass index, etc.) and its functions.
   - they incorporate a function estimation system based on 'random forests' (3).

2. System and apparatus for a non-invasive measurement of glucose levels in blood, according to the previous claim, **characterized in that** the input of the function estimation system (3) consists of a vector of a fixed size including the previous models and information of the patient (sex, age, height, weight, body mass index, pulse rhythm, cardiac coherence, zero-passes of the pre-processed input signal and variability of the zero-passes of the pre-processed input signal etc.).

3.  System and apparatus for a non-invasive measurement of glucose levels in blood, according to the previous claims, **characterized in that** the input signal of the system is a preprocessed plethysmographic wave optically, mechanically or acoustically obtained.

4.  System and apparatus for a non-invasive measurement of glucose levels in blood, according to the previous claims, **characterized in that** the plethysmographic wave has been obtained by means of a digital pulse oximeter.

5.  System and apparatus for a non-invasive measurement of glucose levels in blood, according to the previous claims, **characterized in that** the functions to be estimated in (3) are the basic parameter and linear combinations of these parameter with other parameters of the input vector to decrease the estimation error in (3).

6.  System and apparatus for a non-invasive measurement of glucose levels in blood, according to the previous claims, **characterized in that** it incorporates a post-processing system (4), which performs the average of the system's (3) estimations to decrease the systematic error and the variance of the glucose concentration in blood.

7.  System and apparatus for a non-invasive measurement of glucose levels in blood, according to the previous claims, **characterized in that** the blood glucose level estimation system is implemented by means of devices, for example, by means of FPGA type microcontrollers.

8.  System and apparatus for a non-invasive measurement of glucose levels in blood, according to claim 1, **characterized in that** they comprise a manual device which incorporates at least an acoustic, mechanic and/or optic catheter, comprising inside a data processing system, by means of a CPU, aimed to reduce the variance of the estimated parameter (glucose level in blood).

9.  System and apparatus for a non-invasive measurement of glucose levels in blood, according to the previous claim, **characterized in that** said CPU is implemented by means of DSP, FPGA or microcontroller devices.

10. System and apparatus for a non-invasive measurement of glucose levels in blood, according to claims 8 and 9, **characterized in that** they comprise a storing memory, for example a flash type memory.

11. System and apparatus for a non-invasive measurement of glucose levels in blood, according to claims 8 to 10, **characterized in that** they comprise exterior connecting means to a PC, for example by means of a serial port, Bluetooth or USB (12), and/or network connecting means, for example by means of a WiFi, Zigbee or UWB.

12. System and apparatus for a non-invasive measurement of glucose levels in blood, according claims 8 to 11, **characterized in that** they comprise a data visualizing screen.

13. System and apparatus for a non-invasive measurement of glucose levels in blood, according to claims 8 to 12, **characterized in that** it includes control buttons, batteries and/or connection to an exterior power source.

**FIG.1**

**FIG.2**

**FIG.3**

⑤         ⑥        ⑦

PPG → | H(z) | → | 1/H(z) | → | Statistics |

⑧        ⑨

PPG → | Teager-Kaiser Filter | → | H'(z) |

⑩

PPG → | Cardiac Coherence and Heart Rate | → | Fixed Length Vector V(n) | → Output

⑪

PPG → | Zero-Crossing |

⑫

Patient Data → | Variable Generator |

## FIG.4

PPG(n) → [ z⁻³ ] → [ z⁻³ ] → · · · · → [ z⁻¹ ]

$a_1$      $a_2$      $a_N$

(+) → (+) → · · · · · → (+) → w(n)

## FIG.5

**EP 2 353 506 A1**

## INTERNATIONAL SEARCH REPORT

| International application No. |
| --- |
| PCT/ ES 2009/000529 |

A. CLASSIFICATION OF SUBJECT MATTER

*A61B 5/145* (2006.01)

According to International Patent Classification (IPC) or to both national classification and IPC

B. FIELDS SEARCHED

Minimum documentation searched (classification system followed by classification symbols)

A61B5/00, G01N21

Documentation searched other than minimum documentation to the extent that such documents are included in the fields searched

Electronic data base consulted during the international search (name of data base and, where practicable, search terms used)

INVENES,EPODOC,WPI,INSPEC,NPL,BIOSIS,MEDLINE

C. DOCUMENTS CONSIDERED TO BE RELEVANT

| Category* | Citation of document, with indication, where appropriate, of the relevant passages | Relevant to claim No. |
| --- | --- | --- |
| A | Sheng Lu et al. "A new algorithm for liner and nonliner ARMA modthe parameter estimation using affine geometry", IEEE Transactions on biomedical Engineering, 2001, vol. 48, n° 10, pages 1116-1124 | 1 - 7 |
| A | Chon K H et al. "Robust nonliner autoregressive moving average modthe parameter estimation using stochastic recurrent artificial neural networks", Proceedings of the first joint BMES/ EMBS Conference Serving Humanity, Advancing Technology, October 13-16, ´99, atlanta, GA,USA | 1 - 7 |
| A | El Korek M et al. "Portable integrated multi-signal patient monitoring", 2006 Innovations in information Technology (IEEE Cat. No. 06EX1543C) | 1 - 13 |
| A | Mehdi D et al. "Adaptive control of glucose concentration in diabetic subject´s blood", Modelling and Control in Biomedical Systems, 1989, Pergamon, pages 191 - 195 | 1 - 7 |

☒ Further documents are listed in the continuation of Box C.  ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance.<br>"E" earlier document but published on or after the international filing date | |
| "L" document which may throw doubts on priority claim(s) or which is cited to establish the publication date of another citation or other special reason (as specified)<br>"O" document referring to an oral disclosure use, exhibition, or other means<br>"P" document published prior to the international filing date but later than the priority date claimed | "X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other documents , such combination being obvious to a person skilled in the art |
| | "&" document member of the same patent family |

| Date of the actual completion of the international search | Date of mailing of the international search report |
| --- | --- |
| 22.February.2010      (22.02.2010) | **(01/03/2010)** |
| Name and mailing address of the ISA/<br>O.E.P.M.<br><br>Paseo de la Castellana, 75 28071 Madrid, España.<br>Facsimile No.  34 91 3495304 | Authorized officer<br>A. Cardenas Villar<br><br>Telephone No. +34 91 349 53 93 |

Form PCT/ISA/210 (second sheet) (July 2009)

## INTERNATIONAL SEARCH REPORT

| | International application No. |
|---|---|
| | PCT/ES 2009/000529 |

| C (continuation). | DOCUMENTS CONSIDERED TO BE RELEVANT | |
|---|---|---|
| Category* | Citation of documents, with indication, where appropriate, of the relevant passages | Relevant to claim No. |
| A | El Jabali A K "Development of diabetes mellitus mathematical models from patient´s clinical database", Informatica, July 2004, vol. 28, n° 2, pages 189 - 196 | 1 - 7 |
| A | Clancy N T et al. "Effect of glucose on the optical properties of arterial blood using Mie theory simulations", Proceedings of the SPIE, 2005, vol. 5862, n° 1, pages 58620Y-1-7 | 1 - 7 |
| PA | INSERM et al. "Prediction of the metabolic syndrome status based on dietary and genetic parameters using Random Forest", Genes & Nutrition, December 2008, vol. 3, n° 3-4, pages 173-176 | 1 |
| A | WO 2007141730 A1 (KONINKLIJKE PHILIPS ELECTRONICS) 13.12.2007, the whole document. | 1 - 13 |
| PA | US 7486976 B1 (BELOTSERKOVSKY) 03.02.2009, the whole document. | 1 - 13 |
| A | WO 03063699 A1 (SENSYS MEDICAL INC.) 07.08.2003, the whole document. | 1 - 13 |
| A | US 2005090723 A1 (SAEED) 28.04.2005, the whole document. | 1 - 13 |
| A | WO 03088830 A1 (CAREMATIX INC.) 30.10.2003, the whole document. | 1 - 13 |

Form PCT/ISA/210 (continuation of second sheet) (July 2009)

## INTERNATIONAL SEARCH REPORT

Information on patent family members

| International application No. |
|---|
| PCT/ ES 2009/000529 |

| Patent document cited in the search report | Publication date | Patent family member(s) | Publication date |
|---|---|---|---|
| WO 2007141730 A | 13.12.2007 | EP 2038639 A | 25.03.2009 |
| | | EP 20070736082 | 05.06.2007 |
| | | CN 101460828 A | 17.06.2009 |
| | | US 2009198113 A | 06.08.2009 |
| | | JP 2009539459 T | 19.11.2009 |
| US 7486976 B | 03.02.2009 | US 2009043178 A | 12.02.2009 |
| | | | 12.02.2009 |
| | | | 12.02.2009 |
| WO 03063699 A | 07.08.2003 | CA 2441078 A | 22.08.2002 |
| | | CA 2471564 A | 07.08.2003 |
| | | EP 20020718874 | 25.01.2002 |
| | | US 2004039271 A | 26.02.2004 |
| | | US 2008200783 A | 21.08.2008 |
| | | US 7509153 B | 24.03.2009 |
| | | US 2004068163 A | 08.04.2004 |
| | | US 6990364 B | 24.01.2006 |
| | | US 2004127777 A | 01.07.2004 |
| | | US 7039446 B | 02.05.2006 |
| | | MX P | 15.10.2004 |
| | | EP 1467652 AB | 20.10.2004 |
| | | EP 20030708876 | 24.01.2003 |
| | | US 2004267105 A | 30.12.2004 |
| | | US 7333843 B | 19.02.2008 |
| | | US 2005049466 A | 03.03.2005 |
| | | US 2007060805 A | 15.03.2007 |
| | | US 7233816 B | 19.06.2007 |
| | | JP 2005506517 T | 03.03.2005 |
| | | JP 4071113 B | 02.04.2008 |
| | | CN 1622785 A | 01.06.2005 |
| | | CN 1321610 C | 20.06.2007 |
| | | NZ 527164 A | 29.07.2005 |
| | | AU 2002249985 B | 17.11.2005 |
| | | JP 2006512931 T | 20.04.2006 |
| | | | 20.04.2006 |
| US 2005090723 A | 28.04.2005 | NONE | ----------- |
| WO 03088830 A | 30.10.2003 | CA 2482859 A | 30.10.2003 |
| | | AU 2003223652 A | 03.11.2003 |
| | | US 2004102683 A | 27.05.2004 |
| | | US 7448996 B | 11.11.2008 |
| | | EP 1499233 A | 26.01.2005 |
| | | EP 20030719793 | 15.04.2003 |
| | | US 2009118596 A | 07.05.2009 |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 4704029 A
- WO 5137023 A
- US 5361758 A
- US 6928311 B1
- WO 5383452 A
- WO 5692504 A
- US 6526298 B1
- US 7356364 B1
- US 20010039376 A1
- US 2004F0127779 A1
- US 20030225321 A1
- US 20050070770 A1
- US 20060149142 A1